# FASCICULE DE BREVET EUROPEEN

(11) **EP 2 252 700 B1**
(45) Date de publication et mention de la délivrance du brevet: **17.08.2016**
(21) Numéro de dépôt: 09728585.2
(22) Date de dépôt: 12.03.2009
(51) Int. Cl.: C12Q 1/04

(54) **PROCEDE DE DETECTION EN TEMPS REEL DE MICROORGANISMES DANS UN MILIEU DE CULTURE LIQUIDE PAR LYSE CELLULAIRE**
VERFAHREN ZUM ECHTZEITNACHWEIS VON MIKROORGANISMEN IN EINEM FLÜSSIGEN KULTURMEDIUM MITHILFE ZELLULÄRER LYSE
METHOD FOR REAL-TIME DETECTION OF MICROORGANISMS IN A LIQUID CULTURE MEDIUM USING CELLULAR LYSIS

(30) Priorité: 14.03.2008 FR 0851654
(43) Date de publication de la demande: 24.11.2010
(73) Titulaire: Biomérieux, 69280 Marcy L'etoile (FR)
(72) Inventeur: MOSTICONE, David, F-69280 Sainte Consorce (FR); RAYMOND, Jean-Claude, F-69690 Bessenay (FR); SOFIA, Thierry, F-69290 Saint-Genis-les-Ollières (FR); VIMONT, Antoine, 69005 Lyon (FR)
(86) Numéro de dépôt international: PCT/FR2009/050413
(87) Numéro de publication internationale: WO 2009/122067

(56) Documents cités:
- GASANOV ET AL: "Methods for the isolation and identification of Listeria spp. and Listeria monocytogenes: a review" FEMS MICROBIOLOGY REVIEWS, ELSEVIER, AMSTERDAM, NL, vol. 29, no. 5, 1 novembre 2005 (2005-11-01), pages 851-875, XP005107911 ISSN: 0168-6445
- BLANCO M ET AL: "A TECHNIQUE FOR THE DIRECT IDENTIFICATION OF HEMOLYTIC-PATHOGENIC LISTERIA ON SELECTIVE PLATING MEDIA" LETTERS IN APPLIED MICROBIOLOGY, vol. 9, no. 4, 1989, pages 125-128, XP002500554 ISSN: 0266-8254
- "Microbiology of food and animal feeding stuffs- Horizontal method for the detection and enumeration of Listeria monocytogenes." BRITISH STANDARDS, 1997, pages 1-24, XP002500555
- MOREL P ET AL: "[Transfusion-transmitted bacterial infection: residual risk and perspectives of prevention]" TRANSFUSION CLINIQUE ET BIOLOGIQUE : JOURNAL DE LA SOCIÉTÉ FRANÇAISE DE TRANSFUSION SANGUINE JUN 2003, vol. 10, no. 3, juin 2003 (2003-06), pages 192-200, XP002500556 ISSN: 1246-7820

## Description

Le domaine de l'invention est celui du contrôle microbiologique clinique ou industriel. Plus particulièrement, il s'agit d'une méthode permettant d'identifier un ou plusieurs microorganismes par une réaction de lyse cellulaire, effectuée simultanément à l'enrichissement de l'échantillon en microorganismes.

L'analyse microbiologique nécessite des techniques précises et dont le temps d'obtention du résultat doit être le plus court possible.

Dans le domaine médical, il est nécessaire de prévoir et diagnostiquer le risque infectieux : plus le diagnostic est rapide et précis, plus la prise en charge des malades est efficace et le risque de transmission minimisé. L'approche est similaire pour la santé animale.

Dans le domaine agroalimentaire, le cahier des charges est identique. Il distingue cependant les microorganismes pathogènes et leurs toxines dont la recherche s'applique aux produits commercialisés, les microorganismes non pathogènes, utilisés comme indicateurs-qualité du processus de production, des produits bruts aux produits finis, tout au long de la chaîne, et les bactéries d'intérêt technologique telles les ferments. La détection rapide et précise de contaminations présumées permet de les contrôler et d'engager ainsi des actions correctives.

Techniquement, l'analyse microbiologique peut mettre en oeuvre une ou plusieurs phases de pré-enrichissement/enrichissement, une ou plusieurs phases de détection, une ou plusieurs phases d'énumération des microorganismes. Pour des applications particulières telles le contrôle microbiologique agroalimentaire, une phase de confirmation peut également être requise, afin de répondre aux normes en vigueur dans ce domaine.

La phase de pré-enrichissement/enrichissement fait appel à des milieux de culture, sélectifs ou non, bien connus de l'homme du métier. Des milieux de culture prêts à l'emploi, souvent sous forme liquide, basés sur des formules de milieux traditionnels sont disponibles commercialement.

La phase de détection se base sur la mise en évidence des caractères métaboliques des microorganismes recherchés. On utilise classiquement des substrats enzymatiques spécifiques. Ces substrats enzymatiques sont généralement composés de deux parties, une première partie spécifique de l'activité enzymatique à révéler, appelée également partie cible, et une seconde partie faisant office de marqueur, appelée partie marqueur, généralement constitué par un chromophore ou un fluorophore. Par le choix de ces substrats, selon qu'il y a réaction ou non, il est possible de caractériser la nature d'un microorganisme ou de discriminer différents groupes de microorganismes. Ainsi, l'apparition ou la disparition d'une coloration ou d'une fluorescence sera la signature d'un genre ou d'un type de microorganismes. A cet égard, l'utilisation de milieux chromogènes permet la détection et l'identification simultanées des germes recherchés. Elle simplifie le processus et diminue sensiblement le délai d'obtention du résultat. On citera à titre d'exemple concret les milieux ChromID® de la demanderesse. Ces milieux chromogènes sont basés sur la détection de caractères métaboliques spécifiques des germes recherchés comme par exemple l'activité enzymatique beta-glucuronidase pour *Escherichia coli.* Cependant, certains microorganismes, ou certains sous-types comme par exemple *Escherichia coli* O157:H7, ne présentent pas d'activité enzymatique spécifique et ne peuvent donc pas être détectés à l'aide d'un milieu de culture chromogène.

La phase de confirmation, quant à elle, est plus particulièrement attachée à l'analyse microbiologique dans le domaine agroalimentaire. En effet, lorsque le résultat des méthodes développées précédemment est positif, il est nécessaire de confirmer la présence du pathogène recherché. Ceci impose un test complémentaire et l'utilisation d'un principe de détection différent de celui utilisé lors de la première analyse. Les techniques de biologie moléculaire, basées sur les caractères génomiques des microorganismes recherchés, constituent l'un des moyens utilisés pour confirmer l'identification. On citera à titre d'exemple les techniques d'amplification classiques telles la PCR (Polymerase Chain Reaction) et la NASBA (Nucleic Acid Sequence Based Amplification), qui peuvent être couplées à des techniques de détection en temps réel connues de l'homme du métier.

Les immuno-essais constituent une autre des technologies utilisées pour le test de confirmation. Ils font appel aux caractéristiques immunogènes des microorganismes recherchés. De façon non exhaustive, on peut citer les techniques ELISA (Enzyme Linked ImmunoSorbent Assay), par compétition ou de type sandwich, ou les techniques d'immunoagglutination, détectant des épitopes des microorganismes recherchés. Ces dernières font appel à des supports solides fonctionnalisés, tels des billes (par exemple des particules de latex) recouvertes d'anticorps monoclonaux ou polyclonaux, lesdits supports fonctionnalisés étant mis en contact avec un échantillon biologique, comme l'indiquent par exemple les brevets européens délivrés EP 0 701 624 et EP 1 199 567. Alternativement, comme décrit dans le brevet US-4,659,658, les particules solides peuvent être recouvertes de lectines se liant spécifiquement à des sucres situés à la surface d'un microorganisme donné. Dans tous les cas, l'apparition de l'agglutination permet d'identifier de façon certaine le microorganisme recherché.

L'identification complète et précise d'un microorganisme dans un échantillon nécessite donc l'enchaînement de plusieurs étapes : enrichissement, détection confirmation. Ainsi Blanco et al. (letters in Applied Microbiology 1989, 9, 125-128) décrit une méthode d'identification de *Listeria* hémolytiques pathogènes dans un milieu de culture gélosé avec observation d'une hémolyse après incubation. La standardisation des tests utilisés en routine a permis l'automatisation des méthodes de détection qui restent cependant longues à mettre en oeuvre. Un inconvénient de l'état de la technique est en effet que ces étapes sont réalisées de manière séquentielle. Un autre inconvénient est que la réaction d'interaction spécifique utilisée pour l'étape de confirmation, réaction immunologique ou réaction d'hybridation moléculaire, est, le plus souvent, lue en « point final ». Pendant ce temps, dans l'industrie agroalimentaire, l'intégralité du lot de produit fini est bloquée en attendant le résultat de la confirmation, et en clinique, la mise en place de l'antibiothérapie pertinente et des mesures de prévention est retardée.

Au vu de l'état de la technique considéré, il manque donc un procédé combinant les étapes d'enrichissement, de détection et d'identification précise. Concrètement, un tel procédé conjuguerait rapidité, spécificité, et sensibilité.

La présente invention propose donc de pallier les inconvénients décrits *supra* en mettant en oeuvre simultanément une culture des microorganismes et au moins une réaction de lyse cellulaire par lesdits microorganismes, en milieu liquide.

De façon plus précise, l'invention concerne en premier lieu un procédé de détection d'au moins un microorganisme cible susceptible d'être présent dans un échantillon, comprenant les étapes consistant à :
a) mettre en contact dans un conteneur : un milieu de culture liquide permettant la croissance et la détection du ou des microorganisme(s) cible(s), ledit échantillon et une population cellulaire susceptible d'être lysée par le ou les microorganisme(s) cible(s) ;
b) soumettre l'ensemble à une température favorisant la croissance etla détection du ou des microorganisme(s) cible(s) ;
c) observer en temps réel une lyse de la population cellulaire, indiquant la présence du ou des microorganisme(s) cible(s), ou confirmant ladite présence lorsque lesdits microorganismes cibles sont détectés à l'issue de l'étape b).

Un autre objet de l'invention concerne un procédé d'identification d'au moins un microorganisme cible susceptible d'être présent dans un échantillon, comprenant les étapes consistant à :
a) mettre en contact dans un conteneur : un milieu de culture liquide permettant la croissance et l'identification du ou des microorganisme(s) cible(s), ledit échantillon et une population cellulaire susceptible d'être lysée par le ou les microorganisme(s) cible(s) ;
b) soumettre l'ensemble à une température favorisant la croissance et l'identification du ou des microorganisme(s) cible(s) ; et
c) observer en temps réel une lyse de la population cellulaire dans le milieu de culture liquide, permettant de réaliser l'identification du ou des microorganisme(s) cible(s)ou de confirmer ladite identification, lorsque le ou lesdits microorganismes cible(s) sont identifiés dans ledit milieu de culture, à l'issue de l'étape b).

Un autre objet de la présente invention concerne un procédé d'identification d'au moins un microorganisme cible susceptible d'être présent dans un échantillon, comprenant les étapes consistant à :
a) mettre en contact dans un conteneur : un milieu de culture liquide permettant la croissance et l'identification du ou des microorganisme(s) cible(s), ledit échantillon et une population cellulaire susceptible d'être lysée par le ou les microorganisme(s) cible(s) ;
b) soumettre l'ensemble à une température favorisant la croissance et l'identification des microorganismes ; et
c) observer en temps réel une lyse de la population cellulaire dans le milieu de culture liquide, permettant de compléter l'identification du ou des microorganisme(s), réalisée à l'issue de l'étape b).

Par compléter l'identification, on entend apporter une information supplémentaire permettant de préciser l'identification du ou des microorganisme(s) cible(s). Par exemple, à l'étape a), le milieu de culture utilisé peut être spécifique des bactéries du genre *Escherichia coli* et comporter un substrat spécifique de ce genre bactérien, de sorte que la présence de telles bactéries dans l'échantillon à tester, se caractérise par une modification du milieu de culture, tel un changement de couleur, si le substrat utilisé est un substrat chromogène. La lyse de la population cellulaire (par exemple cellules *Vero*) intervenant à l'étape c) peut, par exemple, permettre de mettre en évidence une souche particulière du genre *Escherichia coli,* telle *E. coli* O157:H7, qui est une souche entéropathogène.

Par population cellulaire, on entend tout type de cellule ou vésicule, d'origine naturelle ou issu du génie chimique ou biologique, susceptible d'être lysé de façon spécifique ou non spécifique par le ou les microorganisme(s) cible(s). Un type de cellule apte à être utilisé dans les procédés selon l'invention est par exemple les érythrocytes ou hématies. En effet, ces cellules sont lysées par les bactéries hémolytiques, en particulier les bactéries β-hémolytiques, telles que certaines souches de *Streptococcus* et *Listeria.* Les hématies utilisées sont susceptibles de contenir des composés aidant à la détection, tel que des composés chromophores ou fluorophores, qui sont libérés lorsque les hématies sont lysées. Une technologie permettant d'encapsuler des composés dans les hématies a été développée par la société ERYtech Pharma®.

Il peut également être envisagé l'utilisation de vésicules lipidiques dont la membrane peut être lysée de façon spécifique ou non par un type de microorganisme. C'est le cas par exemple des liposomes.

La température favorisant la croissance des microorganismes est comprise entre 20 et 44°C et l'échantillon est maintenu à cette température pendant une durée suffisante pour permettre la détection des microorganismes, soit une durée comprise entre 6 et 96 heures.

La mise en oeuvre combinée de ces différentes techniques et dans un conteneur unique permet à la fois de gagner du temps et de limiter les manipulations, donc les contaminations des manipulateurs ou des échantillons, entraînant dans ce dernier cas des faux-positifs. En outre, la mise en oeuvre de l'invention peut être automatisée. On notera également que le gain de temps est lié à la fois à la combinaison de deux étapes en une seule et à la détection de la lyse cellulaire en temps réel.

Avantageusement, les étapes a) et c) des procédés décrits *supra* mettent en oeuvre des composés chromogènes (également dénommés chromophores) ou fluorescents (également dénommés fluorophores).

Plus particulièrement, dans les deux procédés de détection et d'identification, la détection peut préférentiellement mettre en oeuvre l'apparition ou la disparition d'une coloration ou d'une fluorescence. Par ailleurs, dans tous les procédés objets de l'invention, la lyse cellulaire peut avantageusement être mise en évidence par l'apparition ou la disparition d'une coloration ou d'une fluorescence.

Ainsi, selon un mode particulier de réalisation de l'invention, la lyse cellulaire consiste en une hémolyse, à savoir la lyse d'hématies. Un tel mode de réalisation est particulièrement avantageux quand on cherche à mettre en évidence des bactéries hémolytiques. En l'espèce, la réaction d'hémolyse va être mise en évidence par une disparition de la fluorescence. En effet, il s'avère que lors de la lyse des hématies, l'hémoglobine contenue dans ces dernières, se trouve libérée dans le milieu de culture. Elle vient alors masquer la fluorescence émise par les composés fluorogènes contenus dans le milieu de culture.

De façon préférentielle, le conteneur est pris dans le groupe constitué par les microplaques, les microcupules, microtubes, les capillaires ou les cartes multipuits.

Avantageusement, le procédé objet de l'invention peut en outre comporter une étape d'énumération des microorganismes, de préférence selon la méthode du nombre le plus probable explicitée dans le brevet EP 1 105 457 de la Demanderesse.

En outre, l'invention a pour objet un kit de diagnostic permettant la mise en oeuvre du procédé suivant les différents modes de réalisation développés *supra.* Le kit comporte :
- un conteneur pris dans le groupe constitué par les microplaques, les microcupules, les microtubes, les capillaires ou les cartes multipuits;
- un milieu de culture liquide sélectif ou non, ledit milieu de culture contenant ou non un substrat spécifique du métabolisme du genre ou de l'espèce microbienne à détecter ; et
- une population cellulaire susceptible d'être lysée par le genre ou l'espèce mocrobienne à détecter.

Selon un premier mode de réalisation préférentiel, la population cellulaire est constituée d'hématies.

Selon un deuxième mode de réalisation préférentiel, la population cellulaire est constitué de liposomes.

Le kit de diagnostic selon l'invention peut comporter en outre au moins un composé chromogène ou fluorescent.

Enfin, un dernier objet de l'invention concerne l'utilisation d'un kit de diagnostic selon l'invention, pour détecter et/ou identifier au moins un microorganisme susceptible d'être présent dans un échantillon.

L'invention sera mieux comprise à la lecture de la description détaillée qui suit, et des exemples non limitatifs donnés à titre d'illustration.

Le procédé objet de l'invention peut être utilisé pour des échantillons d'origine alimentaire, environnementale ou clinique. L'échantillon est défini comme une petite partie ou petite quantité isolée d'une entité pour l'analyse.

Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

On mentionnera aussi les échantillons liés à l'environnement tels les prélèvements de surface, d'eau, d'air.

Les échantillons d'origine clinique peuvent correspondre à des prélèvements de fluides biologiques (sang total, sérum, plasma, urine, liquide céphalo-rachidien), de selles, des prélèvements de nez, de gorge, de peau, de plaie, d'organes, de tissus ou de cellules isolées etc.

Le contrôle microbiologique correspond à l'analyse d'un échantillon dans le but d'isoler et/ou d'identifier et/ou d'énumérer des microorganismes potentiellement présents tels des bactéries ou des levures. Techniquement, cette analyse comprend la croissance *in vitro* des microorganismes dans un milieu de culture. Par milieu de culture, on entend un milieu comprenant tous les éléments nécessaires à la survie et/ou à la croissance des microorganismes. Le milieu de culture peut contenir d'éventuels additifs comme par exemple : des peptones, un ou plusieurs facteurs de croissance, des hydrates de carbone, un ou plusieurs agents sélectifs, des tampons, un ou plusieurs gélifiants etc. Ce milieu de culture peut se présenter sous forme de liquide, de gel prêt à l'emploi, c'est-à-dire prêt à l'ensemencement en tube, flacon ou sur boite de Pétri.

Au sens de la présente invention, le terme microorganisme recouvre les bactéries gram positif ou gram négatif, les levures, les moisissures et plus généralement, les organismes unicellulaires, invisibles à l'oeil nu, qui peuvent être manipulés et multipliés en laboratoire.

D'une manière générale, le milieu de culture peut en sus contenir un substrat permettant la détection d'une activité enzymatique ou métabolique des microorganismes cibles grâce à un signal détectable directement ou indirectement. Pour une détection directe, ce substrat peut être lié à une partie faisant office de marqueur, fluorescent ou chromogène. Pour une détection indirecte, le milieu de culture selon l'invention peut comporter en sus un indicateur de pH, sensible à la variation de pH induite par la consommation du substrat et révélant la croissance des microorganismes cibles. Ledit indicateur de pH peut être un chromophore ou un fluorophore. On citera comme exemples de chromophores le rouge neutre, le bleu d'aniline, le bleu de bromocresol. Les fluorophores comprennent par exemple la 4-méthylumbelliferone, les dérivés de l'aminocoumarine ou les dérivés de la résorufine.

Au sens de la présente invention, l'identification du microorganisme recherché, potentiellement effectuée par la recherche de ses caractères métaboliques, doit être confirmée. Cette confirmation peut faire appel à des réactions de lyse cellulaire.

Par lyse cellulaire, on entend le résultat d'une action directe ou indirecte des microorganismes sur des cellules ou des vésicules, d'origine naturelle ou non, entraînant une lyse desdites cellules ou une destruction desdites vésicules. La présence dans l'échantillon de bactéries hémolytiques, telles que des streptocoques β-hémolytiques, peut être détectée par la lyse des hématies contenues dans le milieu de culture.

La lyse cellulaire peut être détectée visuellement, ou par un lecteur optique automatique selon différents principes connus de l'homme du métier parmi lesquels on citera, de manière non exhaustive :
(1) la détection de l'apparition d'une fluorescence, par libération de fluorophores lors de la lyse, lesdits fluorophores étant initialement contenus dans les cellules ou vésicules en suspension dans le milieu de culture et ;
(2) la disparition de la fluorescence dans le milieu, due à son masquage par l'hémoglobine libérée par lyse des hématies initialement en suspension dans le milieu de culture ;
(3) la disparition de la fluorescence dans le milieu, due à son masquage par un composé libéré lors de la lyse, ledit composé étant initialement contenu dans les cellules ou vésicules en suspension dans le milieu de culture ;
(4) la détection d'un changement ou d'une apparition de couleur par libération de chromophores, lors de la lyse initialement contenus dans les cellules ou vésicules, initialement en suspension dans le milieu de culture ;
(5) la détection d'un changement de couleur du milieu de culture due à la libération d'hémoglobine par lyse des hématies initialement en suspension dans le milieu de culture.

Les fluorophores utilisables ont été mentionnés *supra* et comprennent la 4-méthylumbelliferone, les dérivés de l'aminocoumarine ou les dérivés de la résorufine.

Les chromophores utilisables sont ceux classiquement utilisés dans les milieux de culture chromogènes et bien connus de l'homme du métier.

Les composés masquant la fluorescence utilisables sont par exemple le paranitrophénol ou l'hémoglobine.

Les opérations de culture/identification puis de lyse cellulaire en point final décrites en deux étapes sont, selon la présente invention, combinées en une seule étape, la lyse cellulaire étant détectée en temps réel. A titre d'illustration, on citera l'identification possible dans un échantillon d'origine alimentaire de *Listeria monocytogenes* : la détection de *Listeria monocytogenes* peut être effectuée à l'aide d'un milieu de culture sélectif de cette bactérie et la confirmation de la présence de *Listeria monocytogenes* sera effectuée simultanément par la réaction de lyse cellulaire, détectée en temps réel. Cette réaction de lyse sera obtenue par exemple en utilisant des vésicules synthétiques, du type liposomes, portant à leur surface des motifs reconnus spécifiquement par l'hémolysine produite par *Listeria monocytogenes,* entraînant ainsi la lyse desdites vésicules.

Dans un mode de réalisation particulier, l'invention peut être mise en oeuvre dans des conteneurs tels des microplaques, des microcupules, des microtubes, des capillaires etc.

Avantageusement, le procédé selon l'invention peut être associé à un dispositif automatique de contrôle microbiologique de type TEMPO® tel que développé par la demanderesse et peut facultativement permettre une énumération des microorganismes détectés.

Le procédé, objet de l'invention, peut être mis en oeuvre à l'aide d'un kit comprenant : un milieu réactionnel contenant une base nutritive, des particules sensibilisées et éventuellement un substrat chromogène spécifique du ou des microorganismes recherchés. Ledit milieu est remis en suspension par un aliquot de l'échantillon à analyser. Avantageusement, le kit permettant de mettre en oeuvre le procédé selon l'invention peut aussi contenir un conteneur solide de type microplaque, microtube, microcupule, capillaire, carte VITEK® ou carte TEMPO®.

### EXEMPLE

### Enumération des Listeria ivanovii basée sur l'utilisation d'hématies d'origine animale

Le test consiste ici en la numération de bactéries *Listeria ivanovii* par voie biochimique en utilisant le système TEMPO®, commercialisé par la demanderesse. Ce test est basé sur un masquage de la fluorescence après lyse spécifique des hématies (libération d'hémoglobine) par *Listeria ivanovii,* issue d'une souche pure (réf. ATCC 70408) initialement présentes dans un ou plusieurs puits de la carte.

### Protocole :

### Etape 1 : remise en suspension du milieu réactionnel par un aliquot de l'échantillon à analyser :

Le milieu réactionnel contient :
- Des hématies d'origine animale (mouton, cheval)
- Un marqueur fluorescent (e.g.7-aminométhylcoumarine (7-AMC), 4-méthylumbélliférone (4-MU))
- Une base nutritive : Eau Peptonnée Tamponnée (référence bioMérieux 51094)

Une suspension bactérienne de *Listeria ivanovii* (1000UFC/ml) est obtenue à partir d'un échantillon de souche pure.

4ml de milieu réactionnel sont inoculés par un aliquote de 40µl de la suspension bactérienne, ce qui correspond à une dilution à 1/100^{ème} de la suspension bactérienne. Le milieu réactionnel est ensuite introduit dans une carte TEMPO®, conformément au protocole en vigueur, en vue de réaliser un dénombrement.

### Etape 2 : incubation de la carte :

Les cartes TEMPO sont ensuite incubées à 37°C pendant 16 à 24h. Au cours de cette période d'incubation, la réaction de lyse des hématies due à la production de listeriolysines (hémolysine produites par les *Listeria ivanovii*) s'effectue simultanément à la croissance bactérienne, en cas de présence de cellules cibles dans le/les puits considérés.

### Etape 3 : lecture du test après 24h d'incubation :

Après incubation, la présence de *Listeria ivanovii,* dans un ou plusieurs puits de la carte, va induire une lyse spécifique des hématies engendrant ainsi une libération d'hémoglobine et donc, un masquage de la fluorescence dans les puits concernés, définis alors comme positifs.

Le nombre de puits positifs est ensuite comptabilisé pour déterminer via la table NPP un nombre d'unités formant colonies (UFC) de *Listeria ivanovii* par gramme ou millilitre d'échantillon.

**Tableau 1 : Unité Relative de Fluorescence (RFU) dans chaque type de puits de la carte TEMPO**

| **Type de puits** | **Nombre de puits positifs** |
|---|---|
| **Grand** | **12** |
| **Moyen** | **8** |
| **Petit** | **1** |

A partir du nombre de puits positifs obtenu, on détermine par la méthode du Nombre le Plus Probable (NPP), la concentration en bactéries de la suspension introduite dans la carte. Ce calcul est réalisé par l'algorithme chargé dans l'instrument TEMPO®. La valeur obtenue est de 10 CFU/ml. Sachant que la suspension bactérienne avait été diluée au 1/100^{ème} dans le milieu réactionnel, on retrouve la concentration de départ de cette dernière qui est de 1000CFU/ml.

## Revendications

1. Procédé de détection d'au moins un microorganisme cible susceptible d'être présent dans un échantillon, comprenant les étapes consistant à :
a. mettre en contact dans un conteneur : un milieu de culture liquide permettant la croissance et la détection du ou des microorganisme(s) cible(s), ledit échantillon et une population cellulaire susceptible d'être lysée par le ou les microorganisme(s) cible(s) ;
b. soumettre l'ensemble à une température favorisant la croissance et la détection du ou des microorganisme(s) cible(s) ;
c. observer en temps réel une lyse de la population cellulaire dans le milieu de culture liquide, indiquant la présence du ou des microorganisme(s) cible(s), ou confirmant ladite présence lorsque lesdits microorganismes cibles sont détectés à l'issue de l'étape b).

2. Procédé d'identification d'au moins un microorganisme cible susceptible d'être présent dans un échantillon, comprenant les étapes consistant à :
a) mettre en contact dans un conteneur : un milieu de culture liquide permettant la croissance et l'identification du ou des microorganisme(s) cible(s), ledit échantillon et une population cellulaire susceptible d'être lysée par le ou les microorganisme(s) cible(s) ;
b) soumettre l'ensemble à une température favorisant la croissance et l'identification du ou des microorganisme(s) cible(s) ; et
c) observer en temps réel une lyse de la population cellulaire dans le milieu de culture liquide, permettant de réaliser l'identification du ou des microorganisme(s) cible(s)ou de confirmer ladite identification, lorsque le ou lesdits microorganismes cible(s) sont identifiés dans ledit milieu de culture, à l'issue de l'étape b).

3. Procédé d'identification selon la revendication 2, comprenant les étapes consistant à :
a) mettre en contact dans un conteneur : un milieu de culture liquide permettant la croissance et l'identification du ou des microorganisme(s) cible(s), ledit échantillon et une population cellulaire susceptible d'être lysée par le ou les microorganisme(s) cible(s) ;
b) soumettre l'ensemble à une température favorisant la croissance et l'identification des microorganismes ; et
c) observer en temps réel une lyse de la population cellulaire dans le milieu de culture liquide, permettant de compléter l'identification du ou des microorganisme(s), réalisée à l'issue de l'étape b).

4. Procédé selon l'une des revendications précédentes; dans lequel la détection ou l'identification dans le milieu de culture liquide met en oeuvre l'apparition ou la disparition d'une coloration ou d'une fluorescence.

5. Procédé selon l'une des revendications précédentes, dans lequel la lyse cellulaire est mise en évidence par l'apparition ou la disparition d'une coloration ou d'une fluorescence.

6. Procédé selon l'une des revendications précédentes, dans lequel le conteneur est pris dans le groupe constitué par les microplaques, les microcupules, les microtubes, les capillaires ou les cartes multipuits.

7. Procédé selon l'une des revendications précédentes, comportant en outre une étape d'énumération des microorganismes.

8. Procédé selon l'une des revendications précédentes, dans lequel la réaction de lyse de la population cellulaire met en oeuvre une réaction bactéries hémolytiques - hématies.

9. Kit de diagnostic microbiologique permettant la mise en oeuvre du procédé selon l'une des revendications précédentes, et comprenant :
- un conteneur pris dans le groupe constitué par les microplaques, les microcupules, les microtubes, les capillaires ou les cartes multipuits ;
- un milieu de culture liquide sélectif ou non, ledit milieu de culture contenant ou non un substrat spécifique du métabolisme du genre ou de l'espèce microbienne à détecter ; et
- une population cellulaire susceptible d'être lysée par le genre ou l'espèce microbienne à détecter.

10. Kit de diagnostic selon la revendication précédente, dans lequel la population cellulaire est constituée de cellules ou vésicules naturelles et/ou issues du génie biologique ou chimique.

11. Kit de diagnostic selon l'une des revendications 9 ou 10, comprenant en outre au moins un composé chromogène ou fluorescent.

12. Utilisation d'un kit de diagnostic selon l'une des revendications 9 à 11, pour détecter et/ou identifier au moins un microorganisme susceptible d'être présent dans un échantillon.

## Patentansprüche

1. Verfahren zum Nachweis mindestens eines Ziel-Mikroorganismus, der in einer Probe vorhanden sein kann, indem man in den folgenden Schritten:
a) in einem Behälter Folgendes in Kontakt bringt: ein flüssiges Kulturmedium, das das Wachstum und den Nachweis des Ziel-Mikroorganismus oder der Ziel-Mikroorganismen gestattet, wobei es sich bei der Probe um eine Zellpopulation handelt, die durch den Ziel-Mikroorganismus oder die Ziel-Mikroorganismen lysiert werden kann;
b) das Ganze einer Temperatur aussetzt, die das Wachstum und den Nachweis des Ziel-Mikroorganismus oder der Ziel-Mikroorganismen fördert;
c) in Echtzeit eine Lyse der Zellpopulation in dem flüssigen Kulturmedium beobachtet, die auf das Vorhandensein des Ziel-Mikroorganismus oder der Ziel-Mikroorganismen hinweist oder das Vorhandensein bestätigt, wenn die Ziel-Mikroorganismen am Ende von Schritt b) nachgewiesen worden sind.

2. Verfahren zur Identifikation mindestens eines Ziel-Mikroorganismus, der in einer Probe vorhanden sein kann, indem man in den folgenden Schritten:
a) in einem Behälter Folgendes in Kontakt bringt: ein flüssiges Kulturmedium, das das Wachstum und die Identifikation des Ziel-Mikroorganismus oder der Ziel-Mikroorganismen gestattet, wobei es sich bei der Probe um eine Zellpopulation handelt, die durch den Ziel-Mikroorganismus oder die Ziel-Mikroorganismen lysiert werden kann;
b) das Ganze einer Temperatur aussetzt, die das Wachstum und die Identifikation des Ziel-Mikroorganismus oder der Ziel-Mikroorganismen fördert; und
c) in Echtzeit eine Lyse der Zellpopulation in dem flüssigen Kulturmedium beobachtet, die die Durchführung der Identifikation des Ziel-Mikroorganismus oder der Ziel-Mikroorganismen oder die Bestätigung der Identifikation gestattet, wenn der Ziel-Mikroorganismus oder die Ziel-Mikroorganismen in dem Kulturmedium am Ende von Schritt b) identifiziert worden sind.

3. Verfahren zur Identifikation nach Anspruch 2, bei dem man in den folgenden Schritten:
a) in einem Behälter Folgendes in Kontakt bringt: ein flüssiges Kulturmedium, das das Wachstum und die Identifikation des Ziel-Mikroorganismus oder der Ziel-Mikroorganismen gestattet, wobei es sich bei der Probe um eine Zellpopulation handelt, die durch den Ziel-Mikroorganismus oder die Ziel-Mikroorganismen lysiert werden kann;
b) das Ganze einer Temperatur aussetzt, die das Wachstum und die Identifikation der Mikroorganismen fördert; und
c) in Echtzeit eine Lyse der Zellpopulation in dem flüssigen Kulturmedium beobachtet, die das Vervollständigen der Identifikation des Ziel-Mikroorganismus oder der Ziel-Mikroorganismen, die am Ende von Schritt b) durchgeführt wurde, gestattet.

4. Verfahren nach einem der vorhergehenden Ansprüche, wobei der Nachweis oder die Identifikation in dem flüssigen Kulturmedium das Auftreten oder Verschwinden einer Färbung oder einer Fluoreszenz nutzt.

5. Verfahren nach einem der vorhergehenden Ansprüche, wobei die Zelllyse durch das Auftreten oder Verschwinden einer Färbung oder einer Fluoreszenz nachgewiesen wird.

6. Verfahren nach einem der vorhergehenden Ansprüche, wobei der Behälter aus der Gruppe ausgewählt ist, die aus Mikrotiterplatten, Mikrovertiefungen, Mikroröhrchen, Kapillaren oder Karten mit mehreren Vertiefungen besteht.

7. Verfahren nach einem der vorhergehenden Ansprüche, bei dem man auch einen Schritt der Zählung der Mikroorganismen durchführt.

8. Verfahren nach einem der vorhergehenden Ansprüche, wobei die Reaktion der Lyse der Zellpopulation eine bakterielle hämolytische Reaktion von Erythrozyten verwendet.

9. Mikrobiologisches Diagnostik-Kit, das die Durchführung des Verfahrens nach einem der vorhergehenden Ansprüche gestattet und Folgendes umfasst:
- einen Behälter, ausgewählt aus der Gruppe, bestehend aus Mikrotiterplatten, Mikrovertiefungen, Mikroröhrchen, Kapillaren oder Karten mit mehreren Vertiefungen;
- ein selektives oder nicht selektives flüssiges Kulturmedien, wobei das Kulturmedium ein für den Metabolismus der nachzuweisenden Mikrobengattung oder -spezies spezifisches Substrat enthält oder nicht; und
- eine Zellpopulation, die durch die nachzuweisende Mikrobengattung oder -spezies lysiert werden kann.

10. Diagnostik-Kit nach dem vorhergehenden Anspruch, wobei die Zellpopulation aus Zellen oder Vesikeln besteht, die natürlich und/oder durch biologische oder chemische Technik entstanden sind.

11. Diagnostik-Kit nach einem der Ansprüche 9 oder 10, das ferner mindestens eine chromogene oder fluoreszierende Verbindung umfasst.

12. Verwendung eines Diagnostik-Kits nach einem der Ansprüche 9 bis 11 zum Nachweis und/oder zur Identifikation von mindestens einen Mikroorganismus, der in einer Probe vorhanden sein kann.

## Claims

1. A method for detecting at least one target microorganism that may be present in a sample, comprising the steps of:
a) bringing into contact, in a container: a liquid culture medium that enables the growth and detection of the target microorganism(s), said sample and a cell population capable of being lysed by the target microorganism(s);
b) subjecting the whole to a temperature that promotes the growth and detection of the target microorganism(s);
c) observing, in real time, lysis of the cell population in the liquid culture medium, indicating the presence of the target microorganism(s) or confirming said presence when said target microorganisms are detected when step b) has been completed.

2. A method for identifying at least one target microorganism that may be present in a sample, comprising the steps of:
a) bringing into contact, in a container: a liquid culture medium that enables the growth and identification of the target microorganism(s), said sample and a cell population capable of being lysed by the target microorganism(s);
b) subjecting the whole to a temperature that promotes the growth and/or identification of the target microorganism(s); and
c) observing, in real time, lysis of the cell population in the liquid culture medium, making it possible to identify the target microorganism(s) or to confirm said identification, when said target microorganism(s) is (are) identified in said culture medium, when step b) has been completed.

3. A method for identifying as claimed in claim 2, comprising the steps of:
a) bringing into contact, in a container: a liquid culture medium that enables the growth and identification of the target microorganism(s), said sample and a cell population capable of being lysed by the target microorganism(s);
b) subjecting the whole to a temperature that promotes the growth and identification of the microorganisms; and
c) observing, in real time, lysis of the cell population in the liquid culture medium making it possible to supplement the identification of the microorganism(s), made when step b) has been completed.

4. The method as claimed in one of the preceding claims, in which the detection or the identification in the liquid culture medium uses the appearance or the disappearance of a coloration or of a fluorescence.

5. The method as claimed in one of the preceding claims, in which the cell lysis is demonstrated by the appearance or the disappearance of a coloration or of a fluorescence.

6. The method as claimed in one of the preceding claims, in which the container is taken from the group constituted of microplates, microcupules, microtubes, capillaries or multiwell cards.

7. The method as claimed in one of the preceding claims, also comprising a step of counting the microorganisms.

8. The method as claimed in one of the preceding claims, in which the reaction of lysis of the cell population employs a hemolytic bacteria - red blood cell reaction.

9. A microbiological diagnostic kit for carrying out the method as claimed in one of the preceding claims, and comprising:
- a container taken from the group constituted of microplates, microcupules, microtubes, capillaries or multiwell cards;
- a selective or nonselective liquid culture medium, said culture medium optionally containing a substrate specific for the metabolism of the microbial genus or species to be detected; and
- a cell population capable of being lysed by the microbial genus or species to be detected.

10. The diagnostic kit as claimed in the preceding claim, in which the cell population is constituted of natural cells or vesicles and/or cells or vesicles derived from biological or chemical engineering.

11. The diagnostic kit as claimed in one of claims 9 or 10, also comprising at least one chromogenic or fluorescent compound.

12. The use of a diagnostic kit as claimed in one of claims 9 to 11, for detecting and/or identifying at least one microorganism that may be present in a sample.
